Europäisches Patentamt

European Patent Office

Office européen des brevets

(19)

(11) **EP 0 531 856 B1**

(12) **EUROPÄISCHE PATENTSCHRIFT**

(45) Veröffentlichungstag und Bekanntmachung des Hinweises auf die Patenterteilung:
**24.07.1996 Patentblatt 1996/30**

(51) Int. Cl.$^6$: **A61M 1/10**, A61M 1/12, F04B 9/02, F04B 43/02

(21) Anmeldenummer: 92114899.5

(22) Anmeldetag: **01.09.1992**

(54) **Pulsierend arbeitende Blutpumpe**

Pulsating blood pump

Pompe à sang pulsatoire

(84) Benannte Vertragsstaaten:
**AT CH DE FR GB IT LI NL SE**

(30) Priorität: **10.09.1991 DE 4129970**

(43) Veröffentlichungstag der Anmeldung:
**17.03.1993 Patentblatt 1993/11**

(73) Patentinhaber: **Forschungsgesellschaft für Biomedizinische Technik e.V.**
**52062 Aachen (DE)**

(72) Erfinder:
• **Kaufmann, Ralf, Dipl.-Ing.**
**W-5100 Aachen (DE)**
• **Reul, Helmut, Prof. Dr. Ing.**
**W-5160 Düren (DE)**
• **Rau, G., Prof. Dr. rer. nat.**
**W-5100 Aachen (DE)**

• **Bitdinger, Ralf, Dipl.-Ing.**
**F-3820 Herbeys (FR)**

(74) Vertreter: **Selting, Günther, Dipl.-Ing. et al**
**Patentanwälte**
**von Kreisler, Selting, Werner**
**Postfach 10 22 41**
**50462 Köln (DE)**

(56) Entgegenhaltungen:
**EP-A- 0 146 445      DE-A- 3 136 969**
**DE-A- 3 825 086      FR-A-   565 023**
**FR-A- 1 075 346      US-A- 3 848 472**

• **CHIRONIS 'mechanisms, linkages and mechanical control' 17. Juni 1966 , MC GRAW-HILL BOOK COMPANY , V.S.A.**
• **D.K.Backman et al., Trans. Amer. Soc. Int. Organs, 19, 542-547 (1973)**

Printed by Rank Xerox (UK) Business Services
2.12.4/3.4

**Beschreibung**

Die Erfindung geht von einer Blutpumpe gemäß dem Oberbegriff des Patentanspruchs 1 aus.

Pulsierend arbeitende Blutpumpen finden als künstlicher Herzersatz oder als Unterstützungspumpe für das menschliche Herz Anwendung. Blutpumpen, die als Herzersatz, also als Implantat, verwendet werden, müssen extrem verschleißarm gebaut sein und einen möglichst hohen Wirkungsgrad haben, da sie batteriegespeist sind und da Eingriffe in das Gerät oder die Stromversorgung nur operativ möglich sind. Außerdem sollte eine derartige Blutpumpe die Pumpbewegung in Annäherung an das Pumpverhalten des natürlichen Herzens ausführen.

Bekannt ist eine Blutpumpe, bei der die Pumpbewegung durch ein Kurbelgetriebe erzeugt wird (D.K. Backman et al., Trans. Amer. Soc. Int. Organs, 19 542-7 (1973) S. 547). Dabei werden zwei gegenüberliegende Kammern von an Membranen anliegenden Kolben abwechselnd in ihrem Volumen verändert. Ein Doppelkolben ist über das Kurbelgetriebe so angetrieben, daß er lineare Hubbewegungen ausführt. Ein derartiger Antrieb erfordert eine Gleitführung der Kurbellager und ist daher verschleißanfällig. Ferner ist sein Wirkungsgrad gering, weil nur eine geometrische Komponente der Kraft für den Antrieb genutzt wird, bei gleichzeitig zu überwindender Gegenreibung. Wegen des kontinuierlich arbeitenden Motors sind die Zeiten von Hub und Rückhub gleich lang.

Ebenfalls bekannt ist eine doppelt wirkende Nockentrommelgetriebepumpe (S. Takatani et al., IEEE 9th Annual Conference of the Engineering and Medicinal and Biological Society, 1987). Dabei werden zwei gegenüberliegende Kolben synchron auseinander- und wieder zusammenbewegt. Die Kolbenbewegung wird durch zwei gegenläufig wirkende, miteinander in Eingriff stehende Trommeln bewirkt. Die beiden Trommeln werden durch eine auf einer Motorwelle angebrachten Nocke bewegt. Die Motorbewegung ist reversibel, um ein Auseinanderbewegen und Wiederzusammenfahren der Trommeln zu ermöglichen. Dies bedingt einen niedrigen Wirkungsgrad bei erhöhtem Verschleiß. Auch hier sind die Hubzeiten und die Rückhubzeiten einander gleich.

Aus DE-C-33 17 156 ist eine Blutpumpe bekannt, die einen dreikeuligen Kolben aufweist und nach dem Prinzip einer Rotationskolbenpumpe arbeitet. Im Innern des Rotationskolbens ist die aus Elektromotor und Planetengetriebe bestehende Antriebseinheit untergebracht. Der Kolben wird in einem Gehäuse mit trochoidenförmiger Mantelbahn bewegt, das Ansaug- und Ausstoßöffnungen aufweist. Diese Blutpumpe ist energieintensiv, da der Kolben mit seiner verhältnismäßig großen Masse entlang der unrunden Mantelbahn bewegt werden muß. Außerdem sind Systole und Diastole gleich lang, wodurch ein unphysiologischer Ansaugunterdruck mittels vergrößerter Ansaugöffnungen vermieden werden muß.

Aus EP-B-0 146 445 ist eine pulsierend arbeitende Blutpumpe bekannt mit mindestens einer in einem Pumpengehäuse angeordneten Kammer und einer von einem drehenden Motor angetriebenen Betätigungsvorrichtung mit einem Getriebe, das entlang einer Kreisbahn einen Kopplungspunkt bewegt, der einen das Kammervolumen periodisch verändernden Kolben treibt. Um das Verhältnis von Systole und Diastole physiologisch nachzubilden, ist es bei dieser Blutpumpe erforderlich, die Motordrehzahl in Abhängigkeit des von einem Sensor ermittelten Blutdrucks ständig zu variieren.

Der Erfindung liegt die Aufgabe zugrunde, eine pulsierend arbeitende Blutpumpe zu schaffen, die mit einfachen Mitteln den zeitlichen Verlauf der Druckveränderungen des natürlichen Herzens nachbildet, während die Drehzahl des Motors konstant bleibt.

Die Lösung der Aufgabe erfolgt mit den Merkmalen des Patentanspruchs 1.

Die erfindungsgemäße Blutpumpe hat infolge der Kolbensteuerung über eine geschlossene Hypozykloidenbahn asymmetrisches Pumpverhalten, das der physiologischen Aufteilung in eine Blutausstoßphase (Systole) und einer sich daran anschließenden Kammerfüllphase (Diastole) entspricht, wobei die Pumpwirkung stoßartig ist und anschließend eine relativ lange Füllphase eintritt. Jeder Kolben durchläuft ruckfrei hintereinander eine Vorhubphase, eine Rückhubphase und eine stationäre Hubphase. Bei Ausführung dieses Zyklus wird die Drehgeschwindigkeit des Motors konstant gehalten. Das asymmetrische Pumpverhalten wird mit einem einfachen Aufbau erzielt, der eine kompakte und leichtgewichtige Bauweise der Blutpumpe ermöglicht.

Die Vorhubphase, die der Systole entspricht, ist kürzer als die Hälfte der Gesamtdauer der drei Hubphasen. Die Aufteilung der Diastole in Rückhubphase und stationäre Hubphase ermöglicht ein dem natürlichen Herzen angepaßtes Pumpverhalten, da die Rückbewegung des Kolbens schneller ist als die Füllgeschwindigkeit der Kammer. Die Kammerfüllung beginnt während der relativ schnellen Rückhubbewegung des Kolbens und setzt sich in der stationären Hubphase fort. Sie erfolgt ausschließlich durch das venöse System in Abhängigkeit vom Blutdruck des mit der Blutpumpe versehenen Patienten. Da der Kolben nicht fest mit der Kammer verbunden ist, schafft die Rückhubbewegung auch keinen Unterdruck mit einer unerwünschten Saugwirkung in der Kammer. Während der stationären Hubphase erreicht die Kammer ihre vollständige Füllung. Es ist daher wichtig, daß während der stationären Hubphase der Kolben keine größeren Hubbewegungen ausführt. Dafür ist es zweckmäßig, eine konvexe (ballige) Hypozykloidenbahn zu verwenden, bei der die Ecken abgerundet sind.

Bei dem Motor kann es sich z.B um einen drehzahlgeregelten Gleichstrommotor oder einen Piezo-Motor handeln. Mittels Sensoren, die z.B. in oder an einer der Kammern angebracht sind und den Füllzustand oder die Füllgeschwindigkeit der Kammer registrieren, kann ein Regelsignal generiert werden, das zur Drehzahlregelung des Motors verwendet wird. Eine Anpassung der Hubfrequenz an den Durchblutungsbedarf des Patienten ist dann möglich.

Eine bevorzugte Ausführungsform zur Realisierung der geschlossenen dreieckigen Hypozykloidenbahn weist ein Ringrad auf, in dem ein außenverzahntes Umlaufrad umläuft, an dem der den Kolben treibende Kopplungspunkt exzentrisch angeordnet ist. Dieses Umlaufrad ist an einem Kurbelarm gelagert, der vom Motor kontinuierlich drehend angetrieben ist, wobei das Umlaufrad entlang des innenverzahnten Ringrads abläuft, dessen Achse mit der Drehachse des Motors übereinstimmt. Das Maß der Exzentrizität des Kopplungspunktes an dem Umlaufrad bestimmt die Form der Hypozykloidenbahn, auf der sich der Kopplungspunkt bewegt. Um eine geschlossene Hypozykloidenbahn zu erhalten, muß der Durchmesser des Ringrads ein ganzzahliges Vielfaches des Durchmessers des Umlaufrads sein. Um eine dreieckige Hypozykloidenbahn zu erhalten, muß dieses Verhältnis 3:1 betragen. Die Länge des Hubweges ist dann gleich der Differenz der Durchmesser von Ringrad und Umlaufrad. Die Verwendung von Zahnrädern zum Erzeugen der Hypozykloidenbahnen ist konstruktiv einfach zu realisieren. Reibung und Verschleiß sind gering. Die dreieckige Hypozykloidenbahnform kann prinzipiell jedoch auch auf andere Weise realisiert werden, z.B. mit Hilfe einer Kulissenführung.

Vorzugsweise ist der Kolben über eine Stange mit dem Kopplungspunkt verbunden, wobei die Lagerung der Stange an beiden Enden über verschleißarme Gelenke erfolgt.

Da der Kolben selbst nicht entlang einer bestimmten Bahn geführt ist, muß die am Kolben angreifende Stange eine Führung aufweisen. Um die Bewegung des Kopplungspunktes entlang der Hypozykloidenbahn in eine lineare Bewegung des Kolbens umzusetzen, ist eine Führungsvorrichtung vorgesehen, die die Stange derart führt, daß ihr kolbenseitiges Ende ausschließlich geradlinig bewegt wird. Vorzugsweise geschieht dies durch ein Lenkergestänge, das am Gehäuse abgestützt ist und gelenkig an der Stange angreift und dessen Lenker ebenfalls durch verschleißarme Gelenke miteinander verbunden sind. Die Wahl einer ausschließlich aus Drehgelenken bestehenden Konstruktion hat eine erhöhte Lebensdauer gegenüber einer Schlitzführung zur Folge. Ferner nimmt ein Lenkergestänge in zusammengeklapptem Zustand wenig Platz in Führungsrichtung der Stange ein, so daß der Kolben nahe an das Getriebe zurückgezogen werden kann. Grundsätzlich kann aber auch eine andere Art von Linearführung für das kolbenseitige Ende der Stange verwendet werden.

Für die Verwendung z.B. als selbständig arbeitendes künstliches Herzimplantat ist es vorteilhaft, wenn innerhalb des Pumpengehäuses zwei Kammern angeordnet sind, die die natürlichen Herzkammern ersetzen und jeweils einen eigenen Kolben aufweisen, wobei für jeden Kolben ein eigener entlang einer Hypozykloidenbahn bewegter Kopplungspunkt vorgesehen ist und die beiden Hypozykloidenbahnen konzentrisch, jedoch gegeneinander verdreht, sind.

Zweckmäßigerweise sind die Kammern und ihre Kolben an entgegengesetzten Enden des Getriebes angeordnet. Wählt man die Anordnung der Kolben und der Kammern innerhalb des Pumpengehäuses so, daß die Hubachsen der beiden Kolben einen Öffnungswinkel zwischen etwa 120° und 170° bilden, eröffnet sich die Möglichkeit, die Enden der Kammern und damit ihre Ein- und Ausgänge eng beieinander anzuordnen und sie damit strömungstechnisch und anatomisch günstig zu plazieren.

Anstelle einer gleichzeitig erfolgenden Bewegung beider Kolben ist es vorteilhaft, beide Kolben im wesentlichen gegenphasig zu betreiben. Bei einem Getriebe mit Umlaufrad hat dies den Vorteil, daß für beide Kolben ein einziges Umlaufrad ausreicht. Ferner wird die Stromquelle zeitlich gleichmäßig belastet.

Wenn beide Kopplungspunkte exzentrisch an demselben Umlaufrad angeordnet sind, ergibt sich eine für Implantate besonders wichtige kleinformatige und leichtgewichtige Bauweise ohne sich gegenseitig kreuzende Kolbenstangen. Bei vorzugsweiser Anordnung der beiden Kopplungspunkte mit gleichem Abstand vom Mittelpunkt des Umlaufrades werden zwei kongruente, d.h. gleichgroße und gleichförmige, jedoch gegeneinander konzentrische verdrehte Hypozykloidenbahnen erzeugt, deren gegenseitige Verdrehung vom gegenseitigen Abstand der Kopplungspunkte abhängt.

Durch Verstellen des Ringrades ist es möglich, die Hypozykloidenbahn gegenüber der Hubachse des Kolbens zu verdrehen und dadurch den Hubverlauf zu beeinflussen. Durch Verdrehung des Ringrads kann die stationäre Phase im Hubverlauf kontinuierlich verkürzt werden. Dieser Effekt kann zu einer Volumenausstoßregelung der Blutpumpe genutzt werden. Zur Verdrehung des Ringrads kann eine Stelleinrichtung, z.B. ein selbsthemmendes Schneckenradgetriebe, vorgesehen sein.

Ausführungsbeispiele der Erfindung sind in den Zeichnungen dargestellt. Es zeigen:

Fig. 1    einen Querschnitt durch eine Ausführungsform der Blutpumpe, wobei einige Partien fortgelassen sind,

Fig. 2    eine schematische Darstellung der Kinematik der Blutpumpe,

Fig. 3    ein Hubverlauf-Zeit-Diagramm der Blutpumpe,

Fig. 4    eine dimetrische Explosionsdarstellung der Blutpumpe,

Fig. 5    eine schematische Darstellung der Kinematik einer anderen Blutpumpe mit einem einzigen Kolben und verstellbarem Ringrad,

Fig. 6    ein Hubverlauf-Zeit-Diagramm der Blutpumpe nach Fig. 5.

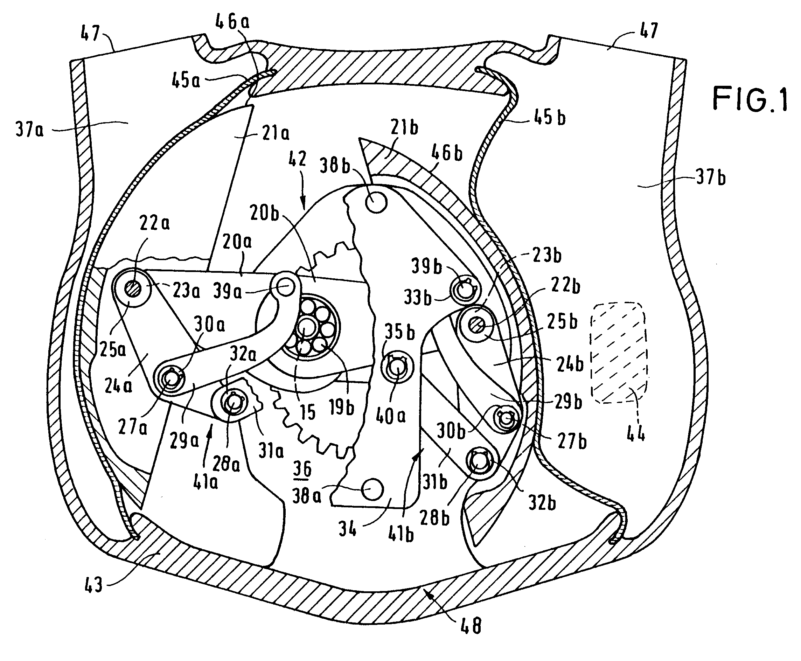

Fig. 1 zeigt einen Querschnitt durch ein Ausführungsbeispiel einer pulsierend arbeitenden Blutpumpe. Einige Partien sind dabei weggeschnitten. In einem Gehäuse 43 sind seitlich zwei Blutkammern 37a und 37b angeordnet, mit gestrichelt eingezeichneten Bluteinlässen 44 und mit Blutauslässen 47. Die Kammern 37a bzw. 37b sind nach innen durch Membranen 45a bzw. 45b begrenzt.

Zwischen den beiden Kammern befindet sich eine Betätigungsvorrichtung 48, die aus einem Getriebe 42, zwei Kolben 21a und 21b mit kalottenartigen Außenflächen 46a und 46b, die an den Membranen 45a und 45b anliegen, und Führungsvorrichtungen 41a und 41b, die als Lenkergestänge ausgebildet sind, besteht. Beim langsamen, selbsttätigen Füllen der Kammern 37a bzw. 37b legt sich die Membran 45a bzw. 45b an die Kolbenaußenfläche 46a bzw. 46b an. Die Membran 45a bzw. 45b führt dann bei weiterer Füllung der Kammer eine Rollfaltenbewegung entlang der Kolben-Außenfläche aus, ohne daß Falten, Überwerfungen oder Beulen in der Membran entstehen.

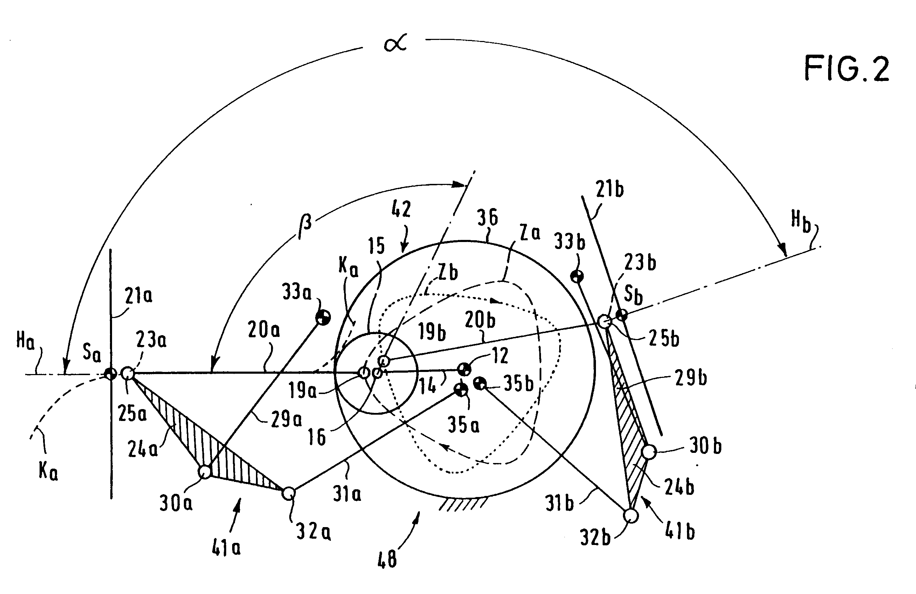

Wie insbesondere aus Fig. 2 ersichtlich ist, ist jeder Kolben 21a und 21b mit dem Getriebe 42 über eine Stange 20a bzw. 20b verbunden, und zwar so, daß der Schwerpunkt $S_a$ bzw. $S_b$ des Kolbens 21a bzw. 21b jeweils im wesentlichen in der Verlängerung dieser Stange liegt. Die Verbindung des Kolbens mit der Stange erfolgt über ein Gelenk 23a bzw. 23b. Durch die Lage der Schwerpunkte Sa bzw. Sb in Bezug auf die Stangen 20a bzw. 20b erfolgt eine dynamische Selbstausrichtung der Kolbenachsen in Richtung der Stangen bzw. der in Fig. 2 gestrichelt eingezeichneten, normal zur Kolbenfläche verlaufenden Hubachsen $H_a$ bzw. $H_b$, wenn der Kolben zurückbewegt wird oder stehenbleibt. Bei der Vorhubbewegung wird der Kolben durch die anliegende Membran ausgerichtet.

Das Getriebe 42 besteht aus einem innenverzahnten Ringrad 36, das fest mit dem Gehäuse 43 verbunden ist, und einem außenverzahnten Umlaufrad 15, das sich gleichmäßig im Ringrad abwälzt. Das Umlaufrad 15 ist an einem Kurbelarm 14 gelagert, der radial von einer koaxial zum Ringrad angeordneten Motorwelle 12 absteht. Die Stange 20a bzw. 20b greift an dem Umlaufrad 15 an einem exzentrisch zur Achse 16 des Umlaufrades angeordneten Kopplungspunkt 19a bzw. 19b an. Bei Drehung des Umlaufrades im Ringrad bewegt sich der Kopplungspunkt 19a bzw. 19b auf einer dreieckigen geschlossenen Hypozykloidenbahn $Z_a$ bzw. $Z_b$. Die Durchmesser von Ringrad und Umlaufrad stehen in einem ganzzahligen Verhältnis, im vorliegenden Fall 3:1, damit die Zykloidenbahn $Z_a$ bzw. $Z_b$ geschlossen ist, d.h. daß bei jedem Umlauf des Umlaufrades stets dieselbe Bahn durchlaufen wird und somit die Eckpunkte dieser Bahn stets an denselben Stellen liegen. Der eine Eckpunkt von $Z_a$ bzw. $Z_b$ ist dem zugehörigen Kolben 21a bzw. 21b zugewandt. Dies wird dadurch erreicht, daß dann, wenn die Achse 16 des Umlaufrades 15 sich auf der durch die Motorwelle 12 und das Gelenk 23a bzw. 23b hindurchgehenden Geraden befindet, auch der Kopplungspunkt 19a bzw. 21b auf dieser Geraden liegt und zwar auf der der Motorwelle 12 abgewandten Seite der Achse 16.

Bei vorgegebenen Durchmessern von Ringrad und Umlaufrad ist das Maß der Exzentrität der Kopplungspunkte entscheidend für die Form der Hypozykloidenbahnen. Es ist für beide Kopplungspunkte identisch und hier so gewählt, daß diese Bahn abgerundete Ecken hat und daß die Seiten der Bahn geringfügig konvex verlaufen.

Mit Ausnahme der gegeneinander verdrehten Hypozykloidenbahnen sind beide Konstruktionen aus Kolben 21a bzw. 21b und Stangen 20a bzw. 20b einander gleich. Diejenigen Teile der Konstruktionen, die einander entsprechen, sind mit denselben Bezugszeichen bezeichnet, wobei lediglich unterschiedliche Zusätze "a" und "b" benutzt wurden.

Lägen die im selben Abstand von der Achse 16 angeordneten beiden Kopplungspunkte 19a und 19b um 180° entgegengesetzt zueinander, wären die zugehörigen Hypozykloidenbahnen genau um 180° gegeneinander verdreht. Die Hubachsen der Kolben würden dann entlang einer einzigen Geraden verlaufen und die Kolbenbewegungen würden exakt abwechselnd erfolgen. Die im wesentlichen senkrecht zu den Hubachsen ausgerichteten Kolbenaußenflächen wären parallel, und dementsprechend auch die Kammern. Die gegenseitige Verdrehung der Hypozykloidenbahnen ist von der gegenseitigen Lage der Kopplungspunkte am Umlaufrad 15 abhängig. Die Gerade durch den Kopplungspunkt 19a und die Achse 16 des Umlaufrades 15 und die Gerade durch den Kopplungspunkt 19b und die Achse 16 definieren einen Öffnungswinkel β. Die Ecken der Hypozykloidenbahn definieren wiederum die Lage der Hubachsen $H_a$ und $H_b$, die einen Öffnungswinkel α einschließen. Die Öffnungswinkel α und β stehen in folgender Beziehung miteinander:

$$\beta = 3 \times (\alpha - 120°)$$

Beim Ausführungsbeispiel bilden die Hubachsen einen Winkel α < 180°. Dadurch können die Auslässe 47 enger zueinander angeordnet sein, was aus anatomischen und strömungstechnischen Gründen wünschenswert ist.

Die Führungsvorrichtung 41a sorgt für die Umsetzung der Bahnbewegung des Kopplungspunkts in einen linearen reversiblen Hub des Kolben 21a. Diese Führungsvorrichtung besteht aus drei Lenkern 24a, 29a, 31a, die gemeinsam ein Lenkergestänge bilden.

Die Lenker 29a und 31a sind jeweils mit einem ersten Ende an gehäusefesten Gelenken 33a und 35a gelagert. Das Gelenk 35a ist in der Nähe der Motorwellenachse 12 angebracht. Das Gelenk 33a ist außerhalb des Umfangs des Ringrads 36 angeordnet, wobei der Lenker 29a die Stange 20a kreuzt. Die Lenker 29a bzw. 31a können Kreispendelbewegungen um die Gelenke 33a bzw. 35a ausführen. Das zweite Ende des Lenkers 31a ist mit einem Lenker 24a über ein Gelenk 32a verbunden. Das zweite Ende des Lenkers 29a ist mit dem Lenker 24a über ein Gelenk 30a verbunden, das im Mittenbereich der Länge des Lenkers 24a angeordnet ist. Über ein Gelenk 25a ist der Lenker 24a an seinem anderen Ende mit der Stange 20a verbunden, wobei die Gelenkachsen der Gelenke 23a und 25a übereinstimmen. Das Gelenk 25a ist durch das Lenkergestänge 24a,29a,31a so geführt, daß es in dem durch die Bewegung des Kopplungspunktes 19a hervorgerufenen Bewegungsabschnitt im wesentlichen lineare Bewegung in Richtung der Hubachse $H_a$ ausführt. Die durch das Lenkergestänge verursachte Bewegungsbahn $K_a$ des Gelenks 25a ist gestrichelt eingezeichnet. Von dieser Bewegungsbahn wird jedoch nur der geradlinige Abschnitt benutzt.

Der Aufbau der Führungsvorrichtung 41b aus einem Lenkergestänge aus den Lenkern 24b,29b,31b sowie den Gelenken 25b,30b,32b,33b,35b ist analog zum Aufbau der Führungsvorrichtung 41a. Jedes Lenkergestänge klappt bei zurückgezogenem Kolben mit geringer Erstreckung in Richtung der Hubachse zusammen. Dadurch kann der Bereich zwischen Getriebe 42 und den zurückgezogenen Kolben klein dimensioniert werden.

Die Funktionsweise des Ausführungsbeispiels wird anhand von Fig. 2 beschrieben. Die kontinuierlich angetriebene Motorwelle 12 führt das um seine Achse 16 drehende Umlaufrad 15 gleichmäßig im Uhrzeigersinn an der Innenwand des Ringrads 36 entlang. Die Kopplungspunkte 19a und 19b bewegen sich auf den Hypozykloidenbahnen $Z_a$ und $Z_b$, die in einer Ebene senkrecht zur Motorwellenachse liegen.

Die Stangen 20a und 20b führen in Abhängigkeit von der Lage des zugehörigen Kopplungspunkts Bewegungen aus, die auf die Kolben 21a und 21b übertragen werden. Die Hubweite ist gleich der Differenz der Durchmesser von Ringrad 36 und Umlaufrad 15. Sie entspricht der Länge der Projektion einer Hypozykloidenbahn, z.B. $Z_a$, auf die zugehörige Hubachse $H_a$. Wenn der Kopplungspunkt die dem Kolben zugewandte, vordere Ecke der Hypozykloidenbahn durchläuft, nimmt der Kolben seine maximale Vorschubstellung ein. Dies ist der Fall für die Stellung des Kolbens 21a in Fig. 2. Beim weiteren Abrollen des Umlaufrades wird der Kolben zurückgezogen, bis der Kopplungspunkt die nächste rückwärtige Ecke der Hypozykloidenbahn erreicht. Der Weg von Ecke zu Ecke der Hypozykloidenbahn entspricht einer Umlaufradumdrehung. Der dem Kolben 21a abgewandte Hypozykloidenbahn-Abschnitt, der nahezu rechtwinklig zur Hubachse verläuft, hat ungefähr die Krümmung eines Kreises, dessen Radius der Länge der Stange 20a zwischen Kopplungspunkt 19a und Gelenk 23a entspricht. Dadurch bewegt sich der Kolben in diesem Bahnabschnitt nur noch ganz gering, während der Kopplungspunkt 19a dieses Kreissegment durchfährt.

In Fig. 2 befindet sich der Kopplungspunkt 19b der Stange 20b auf dem rückwärtigen Abschnitt der Hypozykloidenbahn $Z_b$.

Beim Passieren der dritten Ecke beginnt der Kolben wieder eine Vorhubbewegung, und er erreicht seine maximale Vorschubstellung nach Vollendung der dritten Umlaufradumdrehung.

Aufgrund des Lenkergestänges 24a,29a,31a bzw. 24b,29b,31b kann das außerhalb des Ringrads liegende Ende der Stange 20a bzw. 20b und der mit ihr verbundene Kolben 21a bzw. 21b lediglich eine Bewegung in Richtung der Hubachse $H_a$ bzw. $H_b$ ausführen. Das Lenkergestänge trägt daher das Gewicht von Kolben und Stange. In Abhängigkeit der Vorschubstellung des Kolbens nimmt das Lenkergestänge den zusammengeklappten oder ausgefahrenen Zustand ein.

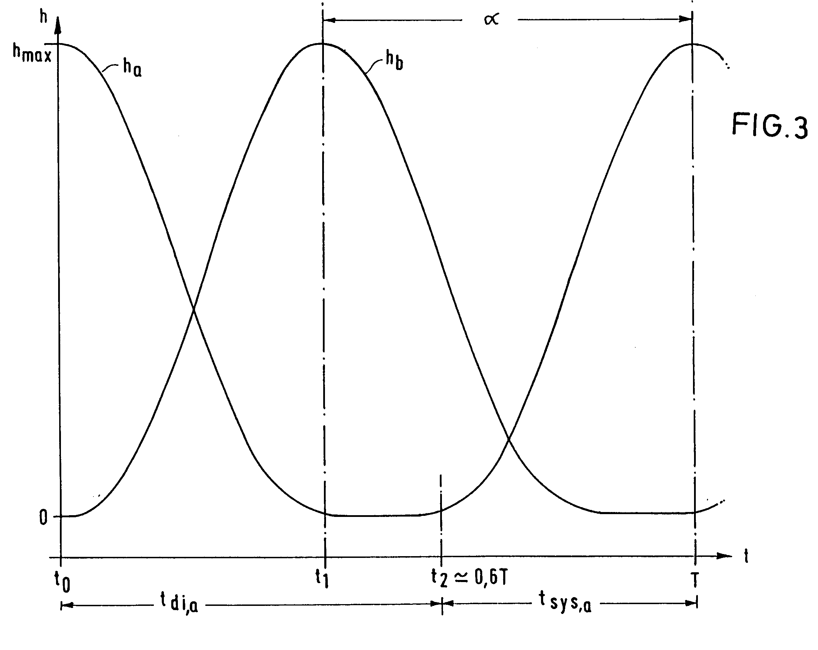

Der Verlauf der Hubbewegungen über der Zeit wird anhand des Diagramms von Fig. 3 erläutert, wobei auf der Abszisse die Zeit t und auf der Ordinate der Kolbenhub h aufgetragen ist. Der Pumpenzyklus erstreckt sich von der Zeit $t_0$ bis nach T. Während des Pumpzyklus durchläuft das Umlaufrad 15 drei Umdrehungen und der Kurbelarm 14 eine volle Umdrehung. Der Wert $h_{max}$ ist der Betrag der maximalen Vorschubstellung eines Kolbens, dessen minimale Vorschubstellung mit h = 0 gesetzt ist.

Die Kolbenpositionen $h_a$ und $h_b$ in Fig. 3 zum Zeitpunkt $t_0$ und zum Zeitpunkt T entsprechen den in Fig. 2 dargestellten Positionen der Kolben 21a bzw. 21b. Der Kolben 21a führt im weiteren Verlauf eine Rückhubphase aus, die bis etwa $t_1 = 0,4$ T dauert und an dieser Stelle den Minimalwert $h_a = 0$ erreicht. Während einer stationären Hubphase bis etwa $t_2 = 0,6$ T behält $h_a$ den Wert Null. In einer anschließenden Vorhubphase erreicht der Kolben 21 wieder seinen Maximalwert $h_a = h_{max}$. Die Systolendauer $t_{sysa}$ beträgt also ca. 40 % der Zykluszeit, während die Diastolendauer $t_{dia}$ etwa 60 % der Zykluszeit ausmacht. Die gleiche Bewegung führt auch der Kolben 21b aus, allerdings um ca. 150° phasenverschoben. Der Phasenwinkel zwischen den beiden Verläufen $h_a(t)$ und $h_b(t)$ ist mit dem Öffnungswinkel $\alpha$ der Hubachsen identisch.

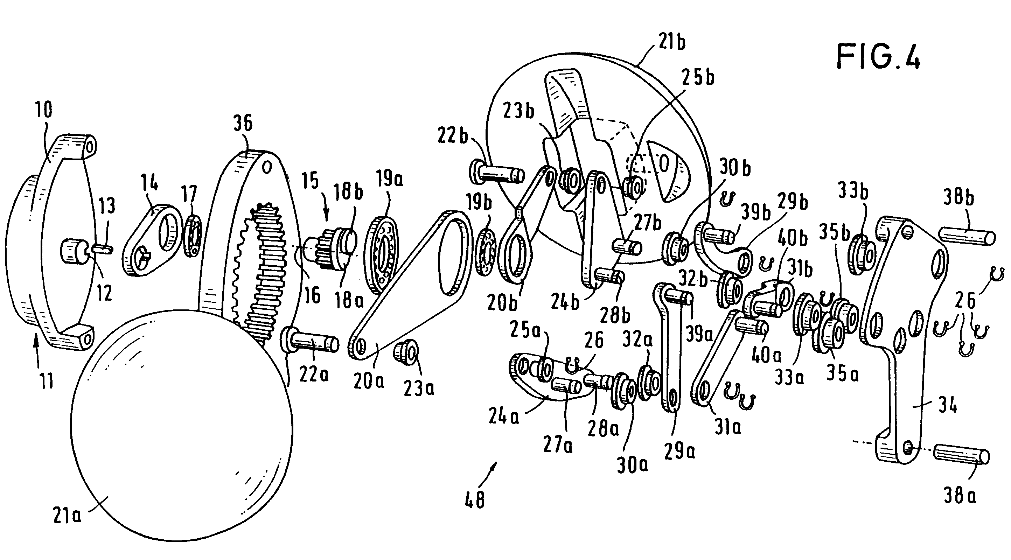

Anhand der Explosionszeichnung in Fig. 4 wird die Zusammensetzung der Blutpumpe aus Fig. 1 und 2 erläutert.

Aus dem Gehäuse 10 eines Motors 11 ragt eine rotierende Welle 12. Mit Hilfe einer Paßfeder 13 ist der Kurbelarm 14 an der Welle 12 befestigt. Das andere Ende des Kurbelarms 14 ist mit dem Umlaufrad 15 über ein Drehlager 17 verbunden. Auf der dem Kurbelarm abgewandten Seite des Umlaufrads 15 sind zwei Wellenstümpfe 18a und 18b angeordnet, deren Mittelpunkte zur Achse 16 des Umlaufrads 15 versetzt sind. Die Kopplungspunkte 19a und 19b werden von Drehlagern gebildet, die an dem einen Ende der Stangen 20a und 20b angebracht sind und die Wellenstümpfe 18a und 18b lagern.

An dem anderen Ende der Stange 20a ist der Kolben 21a mit einem Bolzen 22a und dem Drehgelenklager 23a gelagert. Ebenfalls am Bolzen 22a ist der Lenker 24a mit dem Drehgelenklager 25a gelagert. Der Lenker 24a weist zwei seitliche zylindrische Zapfen 27a und 28a auf. An dem Zapfen 27a ist der Lenker 29a mit dem Drehgelenklager 30a gelagert. An dem Zapfen 28a ist der Lenker 31a mit dem Drehgelenklager 32a gelagert. Die Lenker 29a bzw. 31a weisen an ihrem dem Lenker 24a abgelegenen Ende jeweils zylindrische seitliche Zapfen 39a bzw. 40a auf. Über den Zapfen 39a und dem Drehgelenklager 33a ist der Lenker 29a in einer Halteplatte 34 gelagert. Der Lenker 31a ist mit der Halteplatte 34 über das Drehgelenklager 35a verbunden.

In analoger Bauweise sind die Stange 20b, der Kolben 21b, der Bolzen 22b, die Lenker 24b, 29b, 31b mit den jeweiligen Zapfen 27b, 28b, 39b und 40b sowie Drehgelenklagern 23b, 25b, 30b, 32b, 33b und 35b, sowie mit der Halteplatte 34 verbunden. Die Drehgelenke sind allesamt durch Klemmringe 26 gegen axiale Verschiebungen gesichert.

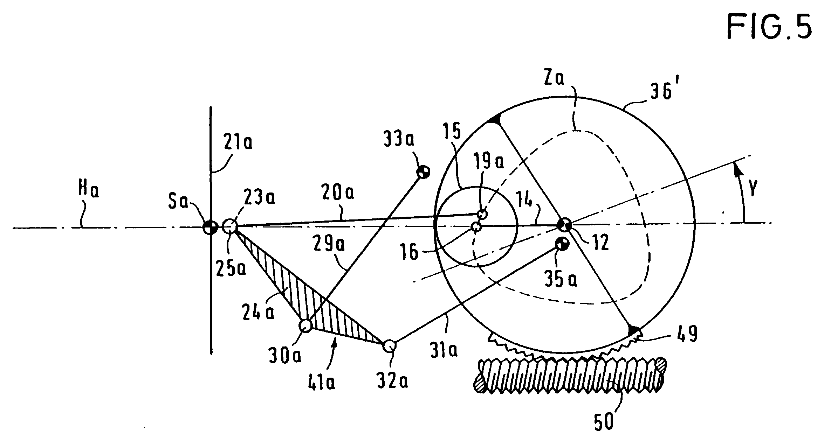

Fig. 5 zeigt eine Blutpumpe mit einem einzigen Kolben 21a und einem verstellbaren Ringrad 36'. Aufbau und Funktionsweise von Getriebe 42, Führungsvorrichtung 41a, Kolben 21a stimmen im übrigen mit dem Ausführungsbeispiel aus Fign. 1-4 überein.

Am äußeren Umfang des Ringrades 36' ist ein Verzahnungssegment 49 angeordnet, das zusammen mit einer Schneckenwelle 50 ein selbsthemmendes Schneckengetriebe bildet. Das Ringrad 36' wird bei Drehung der Schneckenwelle 50 um seine Achse um den Winkel y verdreht, der dann zwischen Hubachse $H_a$ und der vorderen Ecke der Hypozykloidenbahn $Z_a$ gebildet wird.

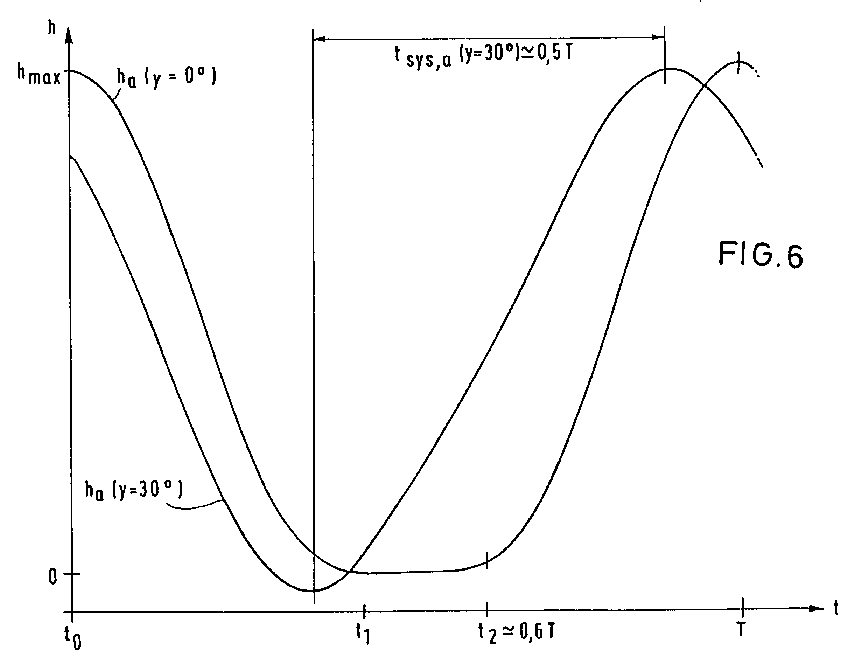

Durch die Verdrehung ändert sich der Hubverlauf h(t) des Kolbens 21a. In Fig. 6 sind die Hubverläufe $h_a(t)$, bei y=0 und $h_a(t)$ bei y=30° in einem Diagramm wie Fig. 3 aufgetragen. Der Hubverlauf $h_a(t)$ bei y=0° entspricht demjenigen aus Fig. 3. Der Hubverlauf $h_a(t)$ bei y=30° hat ein Maximum, das geringfügig kleiner ist als $h_{max}$, und ein Minimum, das ebenfalls kleiner ist als bei y = 0. Die Maxima sind um einen Phasenwinkel gegeneinander verschoben, der etwa dem Winkel y entspricht. Die Rückhub- und die Vorhubphase dauern jetzt ca. 0,5 T, während die stationäre Hubphase entfällt.

Die beschriebene Blutpumpe eignet sich insbesondere für ein künstliches Herzimplantat. Sie ist jedoch auch für den extrakorporalen Einsatz geeignet.

**Patentansprüche**

1. Pulsierend arbeitende Blutpumpe mit mindestens einer in einem Pumpengehäuse (43) angeordneten Kammer (37a,37b) und einer von einem kontinuierlich drehenden Motor (11) angetriebenen Betätigungsvorrichtung (48), die mindestens einen das Kammervolumen periodisch verändernden Kolben (21a,21b) aufweist, sowie ein Getriebe (42), das einen den Kolben (21a,21b) treibenden Kopplungspunkt (19a,19b) entlang einer geschlossenen Bahn bewegt,
   **dadurch gekennzeichnet,**
   daß die Bahn eine dreieckige konvexe Hypozykloidenbahn ($Z_a,Z_b$) ist, deren eine Ecke dem Kolben zugewandt ist.

2. Blutpumpe nach Anspruch 1, dadurch gekennzeichnet, daß die Hypozykloidenbahn ($Z_a,Z_b$) so ausgebildet ist, daß der Kolben (21a,21b) in einem Kolbenzyklus eine Vorhubphase, eine Rückhubphase und eine stationäre Hubphase durchläuft, wobei die Vorhubphase etwa 40 % der Zykluszeit ausmacht.

3. Blutpumpe nach Anspruch 1 oder 2, dadurch gekennzeichnet, daß die Betätigungsvorrichtung ein außenverzahntes Umlaufrad (15) aufweist, das an einem innenverzahnten Ringrad (36) abläuft, wobei der Kopplungspunkt (19a,19b) exzentrisch am Umlaufrad (15) angeordnet ist.

4. Blutpumpe nach Anspruch 3, dadurch gekennzeichnet, daß die Drehstellung des Ringrades (36) veränderbar ist.

5. Blutpumpe nach Anspruch 3 oder 4, dadurch gekennzeichnet, daß das Umlaufrad (15) an einem Kurbelarm (14) gelagert ist, der von dem Motor (11) kontinuierlich drehend angetrieben ist, wobei die Drehachse des Motors (11) mit der Achse des Ringrads (36) übereinstimmt.

6. Blutpumpe nach einem der Ansprüche 1 bis 5, dadurch gekennzeichnet, daß am Kopplungspunkt (19a,19b) eine Stange (20a,20b) drehbar gelagert ist, deren anderes Ende über ein Drehgelenk (23a,23b) mit dem Kolben (21a,21b) verbunden ist.

7. Blutpumpe nach Anspruch 6, dadurch gekennzeichnet, daß eine Führungsvorrichtung (41a) vorgesehen ist, die das kolbenseitige Ende der Stange (20a,20b) entlang einer geradlinigen Bahn führt.

8. Blutpumpe nach Anspruch 7, dadurch gekennzeichnet, daß die Führungsvorrichtung aus einem Lenkergestänge (24a,29a,31a;24b,29b,31b) besteht, das am Pumpengehäuse (43) fest abgestützt ist und gelenkig an der Stange (20a,20b) angreift.

9. Blutpumpe nach einem der Ansprüche 6 bis 8, dadurch gekennzeichnet, daß der Schwerpunkt (5a,5b) des Kolbens (21a,21b) in Verlängerung der Stange (20a,20b) angeordnet ist.

10. Blutpumpe nach einem der Ansprüche 1 bis 9, dadurch gekennzeichnet, daß das Pumpengehäuse zwei Kammern (37a,37b) mit je einem Kolben (21a,21b) enthält, wobei für jeden Kolben (21a,21b) ein eigener entlang einer zugehörigen Hypozykloidenbahn ($Z_a,Z_b$) bewegter Kopplungspunkt (19a,19b) vorgesehen ist und die Hypozykloidenbahnen ($Z_a,Z_b$) konzentrisch, jedoch gegeneinander verdreht sind.

11. Blutpumpe nach Anspruch 10, dadurch gekennzeichnet, daß die beiden Kopplungspunkte (19a,19b) sich auf beiden Hypozykloidenbahnen

($Z_a$,$Z_b$) mit konstantem gegenseitigem Abstand bewegen, der kleiner ist als ein Fünftel des Innenkreisdurchmessers der Hypozykloidenbahnen.

12. Blutpumpe nach Anspruch 10 oder 11, dadurch gekennzeichnet, daß die Hubachsen der beiden Kolben (21a,21b) einen Öffnungswinkel ($\alpha$) zwischen 120° und 170° bilden.

13. Blutpumpe nach einem der Ansprüche 10 bis 12, dadurch gekennzeichnet, daß die Volumenveränderung in den Kammern (37a,37b) des Pumpengehäuses (43) alternierend erfolgt.

14. Blutpumpe nach einem der Ansprüche 10 bis 13, dadurch gekennzeichnet, daß die Betätigungsvorrichtung ein außenverzahntes Umlaufrad (15) aufweist, das an einem innenverzahnten Ringrad (36) abläuft, wobei beide Kopplungspunkte (19a,19b) exzentrisch am Umlaufrad angeordnet sind.

15. Blutpumpe nach Anspruch 14, dadurch gekennzeichnet, daß die beiden Kopplungspunkte (19a,19b) den gleichen Abstand vom Mittelpunkt des Umlaufrades (15) haben.

16. Blutpumpe nach einem der Ansprüche 1 bis 15, dadurch gekennzeichnet, daß der Kolben (21a,21b) eine ellipsoidförmige Außenfläche (46a,46b) besitzt.

**Claims**

1. A blood pump for pulsating operation, comprising at least one chamber (37a,37b) arranged in a pump housing (43) and an operating means (48) driven by a continuously rotating motor (11), said operating means (48) including at least one piston (21a,21b) for periodically changing the chamber volume and a gear unit (42) by which a coupling point (19a,19b) provided for driving the piston (21a,21b) is moved along a closed moving path,
**characterized in**
that said path is a triangular, convex, hypocycloidal path ($Z_a$,$Z_b$) having one of its corners facing towards said piston.

2. The blood pump according to claim 1, characterized in that the hypocycloidal path ($Z_a$,$Z_b$) is arranged in such a manner that, in a piston cycle, the piston (21a,21b) performs an advance stroke period, a return stroke period and a stationary stroke period, the advance stroke period occupying about 40% of the cycle time.

3. The blood pump according to claim 1 or 2, characterized in that the operating means comprises a planet wheel (15) having an outer toothing and moving along an annular wheel (36) having an inner toothing, the coupling point (19a,19b) being arranged eccentrically on the planet wheel (15).

4. The blood pump according to claim 3, characterized in that the rotational position of the annular wheel (36) is variable.

5. The blood pump according to claim 3 or 4, characterized in that the planet wheel (15) is supported on a crank arm (14) rotatingly driven in continuous fashion by the motor (11), the rotational axis of the motor (11) coinciding with the axis of the annular wheel (36).

6. The blood pump according to any one of claims 1 to 5, characterized in that the coupling point (19a,19b) rotatably supports a rod (20a,20b) having its other end connected to the piston (21a,21b) through a pivot joint (23a,23b).

7. The blood pump according to claim 6, characterized in that a guide means (41a) is provided for guiding the piston-side end of the rod (20a,20b) along a rectilinear path.

8. The blood pump according to claim 7, characterized in that the guide means comprises a guide bar arrangement (24a,29a,31a;24b,29b,31b) fixedly supported on the pump housing (43) and pivotably engaging the rod (20a,20b).

9. The blood pump according to any one of claims 6 to 8, characterized in that the center of gravity (5a,5b) of the piston (21a,21b) is located in the extension of the rod (20a,20b).

10. The blood pump according to any one of claims 1 to 9, characterized in that the pump housing comprises two chambers (37a,37b) with one piston (21a,21b) respectively, each of the pistons (21a,21b) having assigned thereto an individual coupling point (19a,19b) moving along a dedicated hypocycloidal path ($Z_a$,$Z_b$), and that the hypocycloidal paths ($Z_a$,$Z_b$) are arranged concentrically but at a rotational displacement from each other.

11. The blood pump according to claim 10, characterized in that the two coupling points (19a,19b) move on both hypocycloidal paths ($Z_a$,$Z_b$) at a constant mutual distance which is smaller than a fifth of the inner circle diameter of the hypocycloidal paths.

12. The blood pump according to claim 10 or 11, characterized in that the stroke axes of the two pistons (21a,21b) define an opening angle ($\alpha$) between 120° and 170°.

13. The blood pump according to any one of claims 10 to 12, characterized in that the change of volume in the chambers (37a,37b) of the pump housing (43) is performed alternately.

**14.** The blood pump according to any one of claims 10 to 13, characterized in that the operating means comprises a planet wheel (15) having an outer toothing and moving along an annular wheel (36) having an inner toothing, both of the coupling points (19a,19b) being arranged eccentrically on the planet wheel.

**15.** The blood pump according to claim 14, characterized in that the two coupling points (19a,19b) are located at the same distance from the center of the planet wheel (15).

**16.** The blood pump according to any one of claims 1 to 15, characterized in that the piston (21a, 21b) has an ellipsoidal outer face (46a,46b).

**Revendications**

**1.** Pompe à sang fonctionnant en mode pulsatoire, comportant au moins une chambre (37a, 37b) disposée dans un carter de pompe (43) et un dispositif de manoeuvre (48) qui est entraîné par un moteur (11) tournant en continu et qui présente au moins un piston (21a, 21b) modifiant périodiquement le volume de la (ou des) chambre(s), ainsi qu'un mécanisme (42) qui déplace le long d'un chemin fermé un point de couplage (19a, 19b) entraînant le piston (21a, 21b),
caractérisée par le fait que
le chemin est un chemin en hypocycloïde convexe triangulaire $(Z_a, Z_b)$ dont un sommet est dirigé vers le piston.

**2.** Pompe à sang selon la revendication 1, caractérisée par le fait que le chemin en hypocycloïde $(Z_a, Z_b)$ est conçu de façon que, dans un cycle du piston, le piston (21a, 21b) parcourt une phase de course aller, une phase de course retour et une phase de course stationnaire, la phase de course aller représentant environ 40% de la durée du cycle.

**3.** Pompe à sang selon l'une des revendications 1 ou 2, caractérisée par le fait que le dispositif de manoeuvre présente une roue rotative à denture extérieure (15) qui roule contre une couronne à denture intérieure (36), le point de couplage (19a, 19b) étant disposé excentriquement sur la roue rotative (15).

**4.** Pompe à sang selon la revendication 3, caractérisée par le fait que la position angulaire de la couronne (36) est modifiable.

**5.** Pompe à sang selon l'une des revendications 3 ou 4, caractérisée par le fait que la roue rotative (15) est portée par un bras de manivelle (14) qui est continuellement entraîné en rotation par le moteur (11), l'axe de rotation du moteur (11) coïncidant avec l'axe de la couronne (36).

**6.** Pompe à sang selon l'une des revendications 1 à 5, caractérisée par le fait qu'au point de couplage (19a, 19b) est portée, avec liberté de rotation, une bielle (20a, 20b) dont l'autre extrémité est reliée au piston (21a, 21b) par l'intermédiaire d'une articulation pivotante (23a, 23b).

**7.** Pompe à sang selon la revendication 6, caractérisée par le fait qu'est prévu un dispositif de guidage (41a) qui guide le long d'un chemin rectiligne l'extrémité de la bielle (20a, 20b) qui est située du côté du piston.

**8.** Pompe à sang selon la revendication 7, caractérisée par le fait que le dispositif de guidage est constitué d'une tringlerie à biellettes (24a, 29a, 31a; 24b, 29b, 31b) qui s'appuie de façon fixe sur le carter de pompe (43) et vient en prise avec la bielle (20a, 20b) par une articulation.

**9.** Pompe à sang selon l'une des revendications 6 à 8, caractérisée par le fait que le centre de gravité (5a, 5b) du piston (21a, 21b) est disposé sur le prolongement de la bielle (20a, 20b).

**10.** Pompe à sang selon l'une des revendications 1 à 9, caractérisée par le fait que le carter de pompe contient deux chambres (37a, 37b) contenant chacune un piston (21a, 21b), un point de couplage propre (19a, 19b), qui se déplace le long d'un chemin en hypocycloïde $(Z_a, Z_b)$ correspondant, étant prévu pour chaque piston (21a, 21b) et les chemins en hypocycloïde $(Z_a, Z_b)$ étant concentriques, mais décalés angulairement l'un par rapport à l'autre.

**11.** Pompe à sang selon la revendication 10, caractérisée par le fait que les deux points de couplage (19a, 19b) se déplacent sur deux chemins en hypocycloïde $(Z_a, Z_b)$ à une distance mutuelle constante qui est inférieure à un cinquième du diamètre du cercle inscrit des chemins en hypocycloïde.

**12.** Pompe à sang selon l'une des revendications 10 ou 11, caractérisée par le fait que les axes de course des deux pistons (21a, 21b) forment un angle d'ouverture ($\alpha$) compris entre 120° et 170°.

**13.** Pompe à sang selon l'une des revendications 10 à 12, caractérisée par le fait que la modification de volume se fait alternativement dans les chambres (37a, 37b) du carter de pompe (43).

**14.** Pompe à sang selon l'une des revendications 10 à 13, caractérisée par le fait que le dispositif de manoeuvre présente une roue rotative à denture extérieure (15) qui roule contre une couronne à denture intérieure (36), les deux points de couplage (19a, 19b) étant disposés excentriquement sur la roue rotative.

15. Pompe à sang selon la revendication 14, caractérisée par le fait que les deux points de couplage (19a, 19b) sont à la même distance du centre de la roue rotative (15).

16. Pompe à sang selon l'une des revendications 1 à 15, caractérisée par le fait que le piston (21a, 21b) possède une surface extérieure (46a, 46b) en forme d'ellipsoïde.

FIG.1

FIG.2

EP 0 531 856 B1

FIG.3

FIG.4

# FIG.5

FIG.6

EP 0 531 856 B1